**Europäisches Patentamt**

**European Patent Office**

**Office européen des brevets**

(19)

(11) Veröffentlichungsnummer : **0 274 498 B2**

(12) **NEUE EUROPÄISCHE PATENTSCHRIFT**

(45) Veröffentlichungstag der neuen Patentschrift :
**17.08.94 Patentblatt 94/33**

(51) Int. Cl.⁵ : **A61K 9/52**, A61K 9/54

(21) Anmeldenummer : **87904381.8**

(22) Anmeldetag : **29.06.87**

(86) Internationale Anmeldenummer :
**PCT/DE87/00295**

(87) Internationale Veröffentlichungsnummer :
**WO 88/00046 14.01.88 Gazette 88/02**

(54) **ARZNEIMITTEL MIT VERZÖGERTER WIRKSTOFFABGABE.**

(30) Priorität : **02.07.86 DE 3622487**

(43) Veröffentlichungstag der Anmeldung :
**20.07.88 Patentblatt 88/29**

(45) Bekanntmachung des Hinweises auf die
Patenterteilung :
**11.09.91 Patentblatt 91/37**

(45) Bekanntmachung des Hinweises auf die
Entscheidung über den Einspruch :
**17.08.94 Patentblatt 94/33**

(84) Benannte Vertragsstaaten :
**AT BE CH DE FR GB IT LI LU NL SE**

(56) Entgegenhaltungen :
**EP-A- 0 107 557**
**EP-A- 0 142 561**
**EP-A- 0 153 105**
**DE-A- 2 054 488**
**DE-A- 2 813 480**
**DE-A- 3 404 595**
**DE-B-20 104 16**
**GB-A- 2 025 227**
**US-A- 4 572 833**
**Chemical Abstracts, vol. 99, No. 4, 25 July
1983, (Columbus, Ohio, US), Y.F. Leung at al.:
"Microencapsulation of crystalline insulin or
islets of Langerhans: an insulin diffusion study", see page 338, abstract 27977e, & Artif.
Organs 1983, 7(2), 208-12**
**Chemical Abstracts, vol. 105, No. 17, 27 October 1986, (Columbus, Ohio, US), W. Hartmeier
et al.: "Membrane-enclosed alginate beads
containing Gluconobacter cells and molecular
dispersed catalase", see page 564, abstract
151506g, & Biotechnol. Lett. 1986, 8(8), 565-70**
**Moderne Arzneimittel (1980), S. 309, S. 954**
**Pharmazeutische Stoffliste (1988), S. 58, S. 29,
S. 306**
**J. Pharm. Pharmacol. 1984, 36, 145-152**
**P.B. Deasy (1984), S. 21, 22, 149**

(56) Entgegenhaltungen :
**Pharmazeutische Technologie (1978); S., 188,
189**
**D. Werner, Pharma International (2) 84, Seite
70ff**
**W. A. Ritschel, Die Tablette 1966, S. 86-88,
127-133**
**Y.F. Leung, Artificial Organs 7 (2), 208-212,
1983**
**Praktikum des Lack-Dragierens, Jan. 1979,
Röhm Pharma GmbH**
**Europäisches Arzneibuch, Band I, S. 35, 1974**
**Firmenprospekt "Eudragit RL und RS", Röhm
Pharma GmbH 1982**
**Lexikon der Pharmacie 1987, S. 190, 273,
332,352, 401, 464, Georg Thieme Verlag Stuttgart**
**C. Eskilson et. al, Manufacturing Chemist Mär-
z-April 1985**
**The Merck Index 10th Ed.**
**Rote Liste 1980 Präparat 45030**
**The Merck Index 11. Ed, Seite 1421**
**Pharm. Ind. 48(Nr.2),187-192 (1986)**

(73) Patentinhaber : **SCHERING
AKTIENGESELLSCHAFT
D-13342 Berlin (DE)**

(72) Erfinder : **FECHNER, Christian
Jenaer Strasse 27
D-1000 Berlin 31 (DE)**
Erfinder : **HÜMPEL, Michael
Singener Weg 24
D-1000 Berlin 37 (DE)**
Erfinder : **WINDT-HANKE, Fred
Steinkirchener Strasse 34
D-1000 Berlin 26 (DE)**
Erfinder : **TACK, Johannes
Tharsanderweg 42
D-1000 Berlin 20 (DE)**

EP 0 274 498 B2

## Beschreibung

Die Erfindung betrifft Arzneimittel mit verzögerter Wirkstoffabgabe, die als Wirkstoff 4-(3-Cyclopentyloxy-4-methoxy-phenyl)-2-pyrrolidon oder 5-[Hexahydro-5-hydroxy-4-(3-hydroxy-4-methyl-1-octen-6-yn-yl)-2(1H)-pentalenyliden)]-pentansäure und Hilfsstoffe enthalten, sowie ein Verfahren zu deren Herstellung.

Es ist bekannt, daß es in der Regel sehr problematisch ist, aus festen, in Wasser schwer löslichen organischen Wirkstoffen Arzneimittel herzustellen, die den Wirkstoff in ausreichender Menge retardierend freisetzen. Die erfindungsgemäßen Arzneimittel sind so ausgestaltet, daß sie im allgemeinen den Wirkstoff in ausreichender Menge über einen ausreichend langen Zeitraum freigeben. Sie sind dadurch gekennzeichnet, daß der Wirkstoff in oder auf Pellets, welche als Füllstoffe Zuckeralkohole, Monosaccharide. Disaccharide, Polysaccharide, chemisch modifizierte Polysaccharide, Magnesiumoxid, Talkum oder Magnesiumstearat enthalten, in mikrokristalliner oder molekulardisperser Form verteilt ist und daß die Pellets mit einer für den Wirkstoff retardierend durchlässigen Diffusionsmembran umhüllt sind.

Die Pellets haben vorzugsweise einen mittleren Durchmesser von 0,3 bis 3,0 mm und insbesondere einen solchen von 0,7 bis 1,4 mm. Die Dicke der sie umhüllenden Membran ist von der Permeabilität des Wirkstoffs abhängig, sie beträgt in der Regel 0,01 bis 0,5 mm und insbesondere 0,04 bis 0,20 mm.

Die Pellets enthalten neben weiteren Hilfsmitteln Füllstoffe aus der Gruppe Zuckeralkohole wie Mannit, Monosaccharide wie Glucose. Fruktose oder Galactose, Disaccharide wie Saccharose oder Lactose, Polysaccharide wie Amylose Stärke oder Cellulose, chemisch modifizierte Polysaccharide wie Methylcellulose, Magnesiumoxid, Talkum, disperse Kieselsäure oder Magnesiumstearat. Besonders bevorzugte Füllstoffe sind die Disaccharide und Polysaccharide.

Als weitere Hilfsmittel seien beispielsweise die in der Galenik üblicherweise verwendeten Bindemittel wie Stärke, Stärkesirup, mikrokristalline Cellulose (beispielsweise Avicel(R)-Lehman u. Voss u. Co., D-Hamburg) Carboxymethylcellulose, Alginate oder Polyvinylpyrrolidon genannt.

Zusätzlich zu den genannten Hilfsstoffen können die Pellets weitere Hilfsstoffe wie Farbstoffe, Geschmackskorregentien, Weichmachersubstanzen etc. enthalten.

Diese Füllstoffe und weiteren Hilfsstoffe werden entweder mit dem mikrokristallinen Wirkstoff mit einer mittleren Korngröße von maximal 0,1 mm vermischt und unter Zusatz von Wasser in dem Fachmann wohl bekannter Weise zu Pellets verarbeitet oder man besprüht wirkstofffreie Pellets mit einer Lösung des Wirkstoffs in einem leicht flüchtigen Lösungsmittel (wie Dichlormethan, Methanol, Ethanol, Isopropanol oder Mischungen dieser Lösungsmittel) und erhält so Pellets die von einer Schicht des molekulardispersen oder mikrokristallinem Wirkstoffs mit einer Korngröße von maximal 0,1 mm überzogen sind.

Zur Erzielung ausreichender Lösungsgeschwindigkeiten sollte der Wirkstoff in der Regel einen Oberflächenwert von mindestens 20 000 cm$^2$ pro g aufweisen. In Fällen extrem geringer Wasserlöslichkeit ist es günstig, den Wirkstoff in molekulardisperser Verteilung anzuwenden. Dies kann man beispielsweise erreichen, indem man ihn aus einer organischen Lösung mit geeigneten Hilfsstoffen von Polyethylenglycol (z.B. PGE 6000) rekristallisiert.

Die so dargestellten Pellets werden erfindungsgemäß mit einer im Magendarmtrakt unlöslichen für den Wirkstoff retardierend durchlässigen Membran umhüllt. Zur Herstellung der Membran können beispielsweise folgende pharmakologisch unbedenklichen Polymere verwendet werden:

Acrylsäureester, Methacrylsäureester, Copolymere der Acryl- und Methacrylsäureester, Vinylacetate, modifizierte Cellulose-Derivate etc.

Besonders geeignete Polymere zur Herstellung der Membran sind unter anderem Copolymere der Methacrylsäure und Methacrylsäureester mit variabel einstellbarem Gehalt an quartären Ammoniumgruppen, die das Außmaß der Hydrophilität und somit auch der Permeabilität der Polymeren bestimmen.

Typische Vertreter dieser Stoffklasse sind z.B. die von der Firma Röhm-Pharma vertriebenen Acrylharze Eudragit(R)-RL und Eudragit(R)-RS. Diese Polymere weisen ein Ammoniumgruppenverhältnis von ca. 1:20 (Eudragit(R)-RL) und ca. 1:40 (Eudragit(R)-RS) - molares Verhältnis der Ammoniumgruppen zu neutralen Acrylsäureestern - auf. Die Permeabilität der Eudragit(R)-RL/RS Membrane kann durch das Mischungsverhältnis der Komponenten beliebig eingestellt werden. Das für eine gewünschte Freigaberate erforderliche Mischungsverhältnis ist für die einzelnen Wirksubstanzen in bekannter Weise zu ermitteln, es liegt üblicherweise in den Grenzen von 20:80 Gewichtsprozent bis 80:20 Gewichtsprozent Eudragit(R)-RL:Eudragit(R)-RS. Die Parmeabilität der Diffusionsmembran kann zusätzlich noch durch Zugabe von Weichmachersubstanzen (z.B. Carbowachs, Triacetin etc.) und eventuell weiterer Hilfsstoffe wie Talkum, oder Magnesiumstearat als Trenn- und Glättungsmittel beeinflußt werden. Zusätzlich kann man den Polymeren zwecks Verbesserung der Färbung beispielsweise Titandioxid oder Buntpigmente wie Eisenoxidpigmente zusetzen, die ebenfalls einen gewissen Einfluß auf die Permeabilität der Diffusionsmembran haben.

Die Pellets werden beispielsweise mit einer Lösung des Polymeren in einem leicht flüchtigen Lösungsmittel (wie Dichlormethan, Methanol, Ethanol, Isopropanol oder Mischungen dieser Lösungsmittel) besprüht. Durch die Auswahl geeigneter Polymere und Zusätze, durch die Wahl der Menge derselben und somit der Dicke der Diffusionsmembran und durch die Auswahl der geeigneten Korngröße des Wirkstoffs kann die Freisetzungsrate des Wirkstoffs in weiten Grenzen beliebig variiert werden. Die Auswahl der optimalen Parameter muß in Vorversuchen, wie sie dem Fachmann hinlänglich geläufig sind, jeweils ermittelt werden .

Die Pellets können gewünschtenfalls in bekannter Weise zu Tabletten oder Dragees verpreßt werden oder in geeignete Kapseln wie solche aus Hartgelatine abgefüllt werden.

Die 5-[Hexahydro-5-hydroxy-4-(3-hydroxy-4-methyl-1octen-6-ynyl)-2(1H)-pentalenyliden)]-pentansäure (=Iloprost) ist ein Antikoagulanticum.

Das 4-(3-Cyclopentyloxy-4-methoxy-phenyl-2-pyrrolidon (=Rolipram) ist ein Psychopharmakum.

Rolipram-haltige Arzneimittel enthalten in den Membran umhüllten Pellets vorzugsweise 0,5-5,0 Gewichtsprozent Wirkstoff, während die Iloprost-haltigen Arzneimittel in den Membran umhüllten Pelletsa vorzugsweise 0,02 bis 0,5 Gewichtsprozent Wirkstoff enthalten.

Die nachfolgenden Ausführungsbeispiele dienen zur näheren Erläuterung der Erfindung.

Beispiel 1

a) 7,2 kg Neutralpellets einer Körnung von 1,1 bis 1,2 mm "Placebo-Pellets" der Firma Werner, D-2082 Tornesch werden in einem geeigneten Dragierkessel mit einer Lösung von

| 0,18 kg | 4-(3-Cyclopentyloxy-4-methoxy-phenyl)-2-pyrrolidon- (=Rolipram) |
| 0,816 kg | Dichlormethan und |
| 0,294 kg | Methanol besprüht. |

Der Sprühauftrag erfolgt mittels eines Ultraschallsprühkopfes oder eines Druckluftzerstäubers, während der Kessel langsam in einem warmen Luftstrom rotiert.

b)

| 1,0 kg | wirkstoffhaltiger Pellets hergestellt nach Beispiel 1 a) werden mit einer Lösung von |
| 0,10 kg | Eudragit(R)RL und |
| 0,40 kg | Eudragit(R)RS in |
| 0,665 kg | Dichlormethan und |
| 0,285 kg | Isopropylalkohol besprüht. |

Der Sprühprozess erfolgt in einem Wirbelschichtgranulator.

Die so erhaltenen Retardpellets weisen die folgende Zusammensetzung auf:

```
  19,56 mg  Rolipram

 944,48 mg  Neutralpelletanteil

   7,26 mg  Eudragit (R) RL

  28,20 mg  Eudragit (R) RS
───────────────────────────────────
1000,00 mg
```

Die in vitro-Freigabeuntersuchung einer abgeteilten Dosis von 4,78 mg Rolipram nach USP XXI in Phosphatpufferlösung pH 7,3 führte zu folgenden Werten:

| nach 2 Stunden | nach 4 Stunden | nach 8 Stunden |
|---|---|---|
| 11,9 % | 31,7 % | 66,8 % der Dosis. |

Beispiel 2

a)

| 0,779 kg | Lactose DIN 30 |
| 0,195 kg | Avicel(R) PH 101 und |
| 0,025 kg | 4-(3-Cyclopentyloxy-4-methoxy-phenyl)-2-pyrrolidon (=Rolipram), mikro 20 |

werden auf 0,315 mmgesiebt und homogen vermischt. Die Pulvermischung wird mit 0,43 kg Wasser intensiv angefeuchtet, über ein geeignetes Lochscheiben-Granulierwerk gegeben, so daß Rohgranulen von ca. 1,0 mm Strangdurchmesser und ca. 4 bis 10 mm Stranglänge entstehen. Die Rohgranulen werden zu Pellets verrundet und getrocknet.

b)

| | |
|---|---|
| 0,3 kg | wirkstoffhaltiger Pellets nach Beispiel 2 a werden mit einer Lösung von |
| 0,01 kg | Eudragit(R) RL und |
| 0,04 kg | Eudragit(R) RS in |
| 0,665 kg | Dichlormethan und |
| 0,285 kg | Isopropanol besprüht. |

Der Sprühprozeß erfolgt in einem Wirbelschichtgranulator.

Die so erhaltenen Retardpellets weisen die folgende Zusammensetzung auf:

$$
\begin{array}{ll}
23,90 \text{ mg} & \text{Rolipram} \\
744,75 \text{ mg} & \text{Lactose} \\
186,40 \text{ mg} & \text{Avicel}^{(R)} \\
9,00 \text{ mg} & \text{Eudragit}^{(R)} \text{ RL} \\
35,96 \text{ mg} & \text{Eudragit}^{(R)} \text{ RS} \\
\hline
1000,00 \text{ mg}
\end{array}
$$

Die in vitro-Freigabeuntersuchung einer abgeteilten Dosis von 4,78 mg Rolipram nach USP XXI in Phosphatpufferlösung pH 7,3 führte zu folgenden Werten:

| nach 2 Stunden | nach 4 Stunden | nach 8 Stunden |
|---|---|---|
| 3,7 % | 8,4 % | 18,9 % der Dosis. |

c) Zur Herstellung der Arzneimittelspezialitäten werden jeweils 200 mg Pellets in Hartgelatinekapseln abgefüllt.

Beispiel 3

a)

| | |
|---|---|
| 0,800 kg | Lactose DIN 30 |
| 0,200 kg | Avicel(R) PH 101 |
| 0,033 kg | β-Cyclodextrin und |
| 0,0012 kg | 5-[Hexahydro-5-hydroxy-4-(3-hydroxy-4-methyl-1-octen-6-yn-yl)-2(1H)-pentaleniden)]-pentansäure (=Iloprost) werden gesiebt und homogen vermischt. Die Pulvermischung wird mit |
| 0,362 kg | Wasser intensiv angefeuchtet, über ein geeignetes Lochscheiben-Granulierwerk gegeben, so daß Rohgranulen von ca. 1,0 bis 1,5 mm Stangendurchmesser und ca. 4 bis 10 mm Stangenlänge entstehen. Die Rohgranulen werden zu Pellets verrundet und getrocknet. |

b)

| | |
|---|---|
| 0,300 kg | wirkstoffhaltiger Pellets nach Beispiel 3a werden mit einer Lösung von |
| 0,010 kg | Eudragit(R) RL und |
| 0,040 kg | Eudragit(R) RS in |
| 0,665 kg | Dichlormethan und |
| 0,285 kg | Isopropanol besprüht. |

Der Sprühprozeß erfolgt in einem Wirbelschichtgranulator.

Die so hergestellten Reatardpellets enthalten pro Gramm 0,97 mg Iloprost.

Die in-vitro-Freigabe einer abgeteilten Dosis von 0,3 mg Iloprost nach USP XXI in Phosphatpuffer-Lösung von pH 7,5 führte zu folgenden Werten:

| Zeit (Stdn.) | Kummulierte Freigabe in % |
|:---:|:---:|
| 1 | 57,1 % |
| 2 | 99,6 % |
| 3 | 99,6 % |
| 6 | 103,5 % |
| 8 | 99,1 % |

Beispiel 4

a)

| | |
|---|---|
| 0,800 kg | Lactose DIN 30 |
| 0,200 kg | Avicel(R) PH 101 |
| 0,033 kg | β-Cyclodextrin |
| 0,0012 kg | 5-[Hexahydro-5-hydroxy-4-(3-hydroxy-4-methyl-1-octen-6yn-yl)-2(1H)-pentaleniden)]-pentansäure (=Iloprost) werden gesiebt und homogen vermischt. Die Pulvermischung wird mit |
| 0,362 kg | Wasser intensiv angefeuchtet, über ein geeignetes Lochscheiben-Granulierwerk gegeben, so daß Rohgranulen von ca. 1,5 mm Stangendurchmesser und ca. 4 bis 15 mm Stangenlänge entstehen. Die Rohgranulen werden verrundet und getrocknet. |

b)

| | |
|---|---|
| 0,300 kg | Pellets hergestellt nach Beispiel 4 a werden mit einer Lösung von |
| 0,015 kg | Eudragit(R) RL und |
| 0,060 kg | Eudragit(R) RS in |
| 0,998 kg | Dichlormethan und |
| 0,428 kg | Isopropanol besprüht. |

Der Sprühprozeß erfolgt über einen Wirbelschichtgranulator.

Von den so hergestellten Pellets wird eine abgeteilte Dosis von 0,3 mg Iloprost nach USP XXI in Phosphatpuffer-Lösung von pH 7,5 auf ihre in-vitro-Freisetzungsrate untersucht, mit folgenden Ergebnis:

| Zeit (Stdn.) | Kumulierte Freigabe in % |
|:---:|:---:|
| 1 | 6,4 |
| 2 | 30,7 |
| 3 | 54,1 |
| 5 | 80,0 |
| 6 | 87,9 |

## Patentansprüche

1. Arzneimittel mit verzögerter Wirkstoffabgabe enthaltend als Wirkstoff 4-(3-Cyclopentyloxy-4-methoxyphenyl)-2-pyrrolidon oder 5-[Hexahydro-5-hydroxy-4-(3-hydroxy-4-methyl-1-octen-6-yn-yl)-2(1H)-pentalenyliden)]-pentansäure und Hilfsstoffe, dadurch gekennzeichnet, daß der Wirkstoff in oder auf Pellets, welche als Füllstoffe Zuckeralkohole, Monosaccharide, Disaccharide, Polysaccharide, chemisch modifizierte Polysaccharide, Magnesiumoxid, Talkum oder Magnesiumstearat enthalten, in mikrokristalliner oder moleculardisperser Form verteilt ist und daß die Pellets mit einer für den Wirkstoff retardierend durchlässigen Diffusionsmembran umhüllt sind.

2. Arzneimittel mit verzögerter Wirkstoffabgabe enthaltend als Wirkstoff 4-(3-Cyclopentyloxy-4-methoxy-phenyl)-2-pyrrolidon oder 5-[Hexahydro-5-hydroxy-4-(3-hydroxy-4-methyl-1-octen-6-yn-yl)-2(1H)-pentalenyliden)]-pentansäure und Hilfsstoffe, dadurch gekennzeichnet, daß der Wirkstoff in oder auf Pellets, welche als Füllstoffe Zuckeralkohole, Monosaccharide, Disaccharide, Polysaccharide, chemisch modifizierte Polysaccharide, Magnesiumoxid, Talkum oder Magnesiumstearat enthalten, in mikrokristalliner oder moleculardisperser Form verteilt ist und daß die Pellets mit einer für den Wirkstoff retardierend durchlässigen Diffusionsmembran bestehend aus einem Copolymeren von Methacrylsäure und Methacrylsäureester mit variabel einstellbarem Gehalt an quartären Ammoniumgruppen umhüllt sind.

3. Arzneimittel gemäß Patentanspruch 1 und 2, dadurch gekennzeichnet, daß die Pellets einen mittleren Durchmesser von 0,3 bis 3,0 mm haben und von einer 0,01 bis 0,5 mm dicken Diffusionsmembran umhüllt sind.

4. Arzneimittel gemäß Patentanspruch 1 bis 3, dadurch gekennzeichnet, daß die Pellets einen wirkstofffreien Kern haben, auf dessen Oberfläche der Wirkstoff in molekulardisperser oder mikrokristalliner Form mit einer mittleren Korngröße von maximal 0,1 mm aufgetragen ist.

5. Arzneimittel gemäß Patentanspruch 4, dadurch gekennzeichnet, daß die wirkstofffreien Pellets im wesentlichen aus Disacchariden und Polysacchariden bestehen.

6. Arzneimittel gemäß Patentanspruch 1 bis 3, dadurch gekennzeichnet, daß die Pellets im wesentlichen aus einer homogenen Mischung von Disacchariden, Polysacchariden und mikrokristallinem Wirkstoff mit einer mittleren Korngröße von maximal 0,1 mm bestehen.

7. Arzneimittel gemäß Patentanspruch 1 bis 6, dadurch gekennzeichnet, daß die umhüllende Membran aus Acrylharz besteht.

8. Arzneimittel gemäß Patentanspruch 1 bis 7, dadurch gekennzeichnet, daß sie als Wirkstoff 4-(3-Cyclopentyloxy-4-methoxy-phenyl)-2-pyrrolidon enthalten.

9. Arzneimittel gemäß Patentanspruch 8, dadurch gekennzeichnet, daß die Membran umhüllten Pellets 0,5-5,0 Gewichtsprozent Wirkstoff enthalten.

10. Arzneimittel gemäß Patentanspruch 1 bis 9, dadurch gekennzeichnet, daß sie als Wirkstoff 5-[Hexahydro-5-hydroxy-4-(3-hydroxy-4-methyl-1-octen-6-yn-yl)-2(1H)-pentalenyliden)]-pentansäure enthalten.

11. Arzneimittel gemäß Patentanspruch 10, dadurch gekennzeichnet, daß die Membran umhüllten Pellets 0,02 bis 0,5 Gewichtsprozent Wirkstoff enthalten.

12. Arzneimittel gemäß Patentanspruch 1 bis 11, dadurch gekennzeichnet, daß die Pellets zu Tabletten oder Dragees verpreßt sind.

13. Verfahren zur Herstellung von Arzneimitteln mit verzögerter Wirkstoffabgabe gemäß Patentanspruch 1 und 2, dadurch gekennzeichnet, daß man den Wirkstoff molekulardispers oder in mikrokristalliner Form auf die Oberfläche wirkstofffreier Pellets aufbringt oder den mikrokristallinen Wirkstoff mit den Hilfsstoffen gleichmäßig mischt und die Mischung zu Pellets formt und die erhaltenen Pellets anschließend mit einer für den Wirkstoff retardierend durchlässigen Diffusionsmembran umhüllt.

## Claims

1. Medicaments with delayed release of the active ingredient, containing as active ingredient 4-(3-cyclopentyloxy-4-methoxyphenyl)-2-pyrrolidone or 5-[hexahydro-5-hydroxy-4-(3-hydroxy-4-methyl-1-octen-6-ynyl)-2(1H)-pentalenylidene)]-pentanoic acid and adjuvants, characterised in that the active ingredient is distributed in microcrystalline or molecularly dispersed form in or on pellets that contain, as fillers, sugar alcohols, monosaccharides, disaccharides, polysaccharides, chemically modified polysaccharides, magnesium oxide, talcum or magnesium stearate, and the pellets are coated with a permeable diffusion membrane that retards the release of the active ingredient.

2. Medicaments with delayed release of the active ingredient, containing as active ingredient 4-(3-cyclopentyloxy-4-methoxyphenyl)-2-pyrrolidone or 5-[hexahydro-5-hydroxy-4-(3-hydroxy-4-methyl-1-octen-6-ynyl)-2(1H)-pentalenylidene)]-pentanoic acid and adjuvants, characterised in that the active ingredient is distributed in microcrystalline or molecularly dispersed form in or on pellets that contain, as fillers, sugar alcohols, monosaccharides, disaccharides, polysaccharides, chemically modified polysaccharides, magnesium oxide, talcum or magnesium stearate, and the pellets are coated with a permeable diffusion membrane that retards the release of the active ingredient, the diffusion membrane comprising a copolymer of methacrylic acid and methacrylic acid ester having a variably adjustable content of quaternary ammonium groups.

3. Medicaments according to patent claims 1 and 2, characterised in that the pellets have a mean diameter of from 0.3 to 3.0 mm and are coated with a diffusion membrane from 0.01 to 0.5 mm thick.

4. Medicaments according to patent claims 1 to 3, characterised in that the pellets have a core that is free of active ingredient and to the surface of which the active ingredient is applied in molecularly dispersed or microcrystalline form at a mean particle size of no more than 0.1 mm.

5. Medicaments according to patent claim 4, characterised in that the active-ingredient-free pellets comprise essentially disaccharides and polysaccharides.

6. Medicaments according to patent claims 1 to 3, characterised in that the pellets comprise essentially a homogeneous mixture of disaccharides, polysaccharides and microcrystalline active ingredient having a mean particle size of no more than 0.1 mm.

7. Medicaments according to patent claims 1 to 6, characterised in that the coating membrane comprises acrylic resin.

8. Medicaments according to patent claims 1 to 7, characterised in that they contain 4-(3-cyclopentyloxy-4-methoxyphenyl)-2-pyrrolidone as active ingredient.

9. Medicaments according to patent claim 8, characterised in that the membrane-coated pellets contain from 0.5 to 5.0 % by weight active ingredient.

10. Medicaments according to patent claims 1 to 9, characterised in that they contain 5-[hexahydro-5-hydroxy-4-(3-hydroxy-4-methyl-1-octen-6-ynyl)-2(1H)-pentalenylidene)]-pentanoic acid as active ingredient.

11. Medicaments according to patent claim 10, characterised in that the membrane-coated pellets contain from 0.02 to 0.5 % by weight active ingredient.

12. Medicaments according to patent claims 1 to 11, characterised in that the pellets have been compressed to form tablets or dragées.

13. A process for the preparation of medicaments with delayed release of the active ingredient, according to patent claims 1 and 2, characterised in that the active ingredient is applied in molecularly dispersed or microcrystalline form to the surface of active-ingredient-free pellets, or the microcrystalline active ingredient is mixed homogeneously with the adjuvants and the mixture is moulded into pellets and the resulting pellets are then coated with a permeable diffusion membrane that retards the release of the active ingredient.

## Revendications

1. Médicaments à libération retardée du principe actif, contenant en tant que principe actif la 4-(3-cyclopentyloxy-4-méthoxy-phényl)-2-pyrrolidone ou l'acide 5-[hexahydro-5-hydroxy-4-(3-hydroxy-4-méthyl-1-octèn-6-yn-yl)-2(1H)-pentalénylidène)-pentanoïque et des adjuvants, caractérisés en ce que le principe actif est réparti, sous forme microcristalline ou à dispersion moléculaire, dans ou sur des pastilles contenant en tant que charges des alcools de sucre, des monosaccharides, des disaccharides, des polysaccharides, des polysaccharides chimiquement modifiés, de l'oxyde de magnésium, du talc ou du stéarate de magnésium, et que les pastilles sont enrobées d'une membrane de diffusion perméable qui laisse passer le principe actif avec un effet retard.

2. Médicaments à libération retardée du principe actif, contenant en tant que principe actif la 4-(3-cyclopentyloxy-4-méthoxy-phényl)-2-pyrrolidone ou l'acide 5-[hexahydro-5-hydroxy-4-(3-hydroxy-4-méthyl-1-octèn-6-yn-yl)-2(1H)-pentalénylidène)-pentanoïque et des adjuvants, caractérisés en ce que le principe actif est réparti, sous forme microcristalline ou à dispersion moléculaire, sur ou dans des pastilles contenant en tant que charges des alcools de sucre, des monosaccharides, des disaccharides, des polysaccharides, des polysaccharides modifiés chimiquement, de l'oxyde de magnésium, du talc ou du stéarate de magnésium, et que les pastilles sont enrobées d'une membrane de diffusion perméable qui laisse passer le principe actif avec un effet retard, laquelle membrane se compose d'un copolymère d'acide méthacrylique et d'ester de l'acide méthacrylique ayant une teneur réglable de façon variable en groupes ammonium quaternaire.

3. Médicaments selon les revendications 1 et 2, caractérisés en ce que les pastilles ont un diamètre moyen de 0,3 à 3,0 mm et sont enrobées d'une membrane de diffusion d'une épaisseur de 0,01 à 0,5 mm.

4. Médicaments selon les revendications 1 à 3, caractérisés en ce que les pastilles comportent un noyau sans principe actif, sur la surface duquel le principe actif est appliqué, sous forme à dispersion moléculaire ou microcristalline, avec une granulométrie moyenne maximale de 0,1 mm.

5. Médicaments selon la revendication 4, caractérisés en ce que les pastilles sans principe actif se composent essentiellement de disaccharides et de polysaccharides.

6. Médicaments selon les revendications 1 à 3, caractérisés en ce que les pastilles se composent essentiellement d'un mélange homogène de disaccharides, de polysaccharides et d'un principe actif microcristallin ayant une granulométrie moyenne maximale de 0,1 mm.

7. Médicaments selon les revendications 1 à 6, caractérisés en ce que la membrane enrobante se compose de résine acrylique.

8. Médicaments selon les revendications 1 à 7, caractérisés en ce qu'ils contiennent, en tant que principe actif, la 4-(3-cyclopentyloxy-4-méthoxyphényl)-2-pyrrolidone.

9. Médicaments selon la revendications 8, caractérisés en ce que les pastilles enrobées de la membrane ont une teneur en pricipe actif de 0,5 à 5,0 % en poids.

10. Médicaments selon les revendications 1 à 9, caractérisés en ce qu'ils contiennent en tant que principe actif l'acide 5-[hexahydro-5-hydroxy-4-(3-hydroxy-4-méthyl-1-octèn-6-yn-yl)-2(1H)-pentalénylidène)-pentanoïque.

11. Médicaments selon la revendication 10, caractérisés en ce que les pastilles enrobées de la membrane contiennent de 0,02 à 0,5 % en poids de principe actif.

12. Médicaments selon les revendications 1 à 11, caractérisés en ce que les pastilles sont comprimées pour obtenir des comprimés ou dragées.

13. Procédé pour la préparation de médicaments à libération retardée au principe actif selon les revendications 1 et 2, caractérisé en ce qu'on applique le principe actif, sous forme à dispersion moléculaire ou microcristalline, sur la surface de pastilles sans principe actif, ou que l'on mélange jusqu'à l'homogénéité le principe actif avec des adjuvants, et on façonne le mélange pour obtenir des pastilles et ensuite on enrobe les pastilles obtenues à l'aide d'une membrane de diffusion perméable qui laisse passer le principe actif avec un effet retard.